# EUROPEAN PATENT APPLICATION

(11) **EP 3 546 022 A1**
(43) Date of publication of application: **02.10.2019**
(21) Application number: 19152822.3
(22) Date of filing: 21.01.2019
(51) Int. Cl.: A61N 5/10

(54) **PARTICLE THERAPY PLANNING APPARATUS, PARTICLE THERAPY SYSTEM, AND DOSE DISTRIBUTION CALCULATION PROGRAM**

(30) Priority: 29.03.2018 JP 2018063560
(71) Applicant: Hitachi, Ltd., Tokyo 100-8280 (JP)
(72) Inventor: FUJII, Yusuke, Tokyo, 100-8280 (JP); FUJITAKA, Shinichiro, Tokyo, 100-8280 (JP)
(74) Representative: MERH-IP Matias Erny Reichl Hoffmann Patentanwälte PartG mbB

(57) **Abstract**

To improve calculation accuracy of a dose distribution regarding a moving target in continuous scanning irradiation that irradiates a particle beam even during an irradiation position change. A therapy planning apparatus 501 is configured to: discretize an irradiation amount with which a particle beam is irradiated during an irradiation position change for calculation; associate a series of 3D CT images included in a 4D CT image with elapsed time information from a start to a completion of irradiation of a particle beam 300; distribute the irradiation amount discretized for the calculation to the 3D CT images based on the 3D CT images and the elapsed time information that are associated; calculate a dose distribution of the particle beam 300 on the 3D CT images to which the irradiation amount is distributed; calculate corresponding positions between the 3D CT images based on non-rigid registration; and integrate a dose distribution formed for each of the 3D CT images during an irradiation period for each of the corresponding positions between the 3D CT images from the start to the completion of the irradiation of the particle beam 300.

## Description

### Technical Field

The present invention relates to a particle therapy planning apparatus, a particle therapy system, and a dose distribution calculation program that are capable of calculating a dose distribution of particle beams with which a target volume is to be irradiated.

### Background Art

Radiotherapy for the purpose of necrotizing tumor cells by irradiating with various radioactive rays has been widely used in recent years. A therapy is widespread that uses particle beams including not only the most widely used X-rays but also proton beams and carbon ion beams as the radioactive rays used for the radiotherapy.

In a particle therapy, the use of a scanning method is widespread. The scanning method is a method in which a high dose of fine particle beams are applied only to a tumor region by irradiating the fine particle beams so as to fill the inside of a tumor. The scanning method can form a variety of dose distributions without essentially the need for a patient specific device, such as a collimator that shapes the dose distribution into a tumor shape.

In the particle therapy, it is necessary to previously make a detailed therapy plan for a state of a position and a target volume to be irradiated with the particle beams. An irradiation amount and an irradiation position are previously determined by a therapy planning apparatus so as to obtain a desired dose distribution for the target volume or an area surrounding the target volume. It is most common to use X-ray CT (Computed Tomography) images (hereinafter referred to as CT images) to verify a state inside a body of a patient during a prior therapy plan. Target position designation and inside-body dose distribution calculation based on the designation thereof are often performed using the CT images.

Although it is desired that a planned irradiation is performed during irradiation, errors caused by various factors actually occur. The factors for the errors include a target volume movement due to respiration or heartbeat during irradiation in addition to an error caused by the apparatus itself and an error during positioning. It is difficult to make an evaluation since the target volume movement such as respiration or heartbeat varies from patient to patient and from target position to target position. In scanning irradiation, since a desired dose distribution is formed by superimposing complex dose distributions, it is particularly difficult to predict a change in dose distribution with respect to a movement such as respiration.

For the purpose of quantitatively evaluating an influence of the target volume movement on the dose distribution, a method of predicting a movement of an organ around the target volume by making CT images having time-changing information may be considered. A CT image having time information is called a four-dimensional CT image (hereinafter referred to as a 4D CT image). The 4D CT image holds, from images in a state within a short time frame with respect to a moving subject, a set of three-dimensional CT images (hereinafter, referred to as 3D CT images) imaged at different time points.

For example, normal CT images are images that are time-averaged over a respiratory cycle regarding a respiratory movement. In contrast, the 4D CT image is a set of 3D CT images imaged at certain time points during a cycle of an inhaling state or an exhaling state.

There is a method of predicting the influence of the target volume movement on the dose distribution calculation by the therapy planning apparatus using the 4D CT image. PTL 1 discloses a method of calculating a dose distribution when a therapy is performed in a state of a movement represented by a 4D CT image. In this method, the 4D CT image is associated with elapsed time information from a start to a completion of a particle therapy based on a therapy plan. Thereafter, based on the elapsed time information and the 4D CT image, an irradiation amount of a particle beam for irradiation positions are divided and allocated to 3D CT images of each phase, so as to calculate and then integrate dose distributions. The energy and irradiation amount for each of the irradiation positions are determined. In a discrete scanning irradiation in which particle beam irradiation is stopped when the irradiation position is changed, dose distribution calculation on the 4D CT image can be accurately performed by the method described in PTL 1.

### Prior Art Literature

### Patent Literature

PTL 1: JP-A-2014-42815

### Summary of Invention

### Technical Problem

However, in continuous scanning irradiation in which the particle beam is irradiated even during an irradiation position change, there is also a dose irradiated between predetermined irradiation positions. In the above method, since the dose during the irradiation position change is approximated as a dose of the predetermined irradiation position, it is difficult to accurately calculate the dose distribution on the 4D CT image.

The invention is made in view of the above circumstances . An object of the invention is to provide a particle therapy planning apparatus, a particle therapy system, and a dose distribution calculation program that are capable of improving calculation accuracy of a dose distribution regarding a moving target in continuous scanning irradiation in which a particle beam is irradiated even during an irradiation position change.

### Solution to Problem

In order to achieve the above object, a particle therapy planning apparatus according to a first aspect is configured to calculate an irradiation amount of a particle beam during an irradiation position change in continuous scanning irradiation, and to calculate a dose distribution of the particle beam with respect to a moving target based on the irradiation amount of the particle beam during the irradiation position change.

### Advantageous Effect

According to the invention, it is possible to improve calculation accuracy of a dose distribution regarding a moving target in continuous scanning irradiation in which a particle beam is irradiated even during an irradiation position change.

### Brief Description of Drawings

[Fig. 1] Fig. 1 is a block diagram showing a configuration of a particle therapy system to which a particle therapy planning apparatus is applied according to an embodiment.
[Fig. 2] Fig. 2 is a block diagram showing configurations of an irradiation field forming apparatus and a power supply apparatus of Fig. 1.
[Fig. 3] Fig. 3 is a perspective view showing an example of setting an irradiation position in continuous scanning irradiation according to the embodiment.
[Fig. 4] Fig. 4 is a perspective view showing an example of setting an irradiation position when energy is changed in the continuous scanning irradiation according to the embodiment.
[Fig. 5] Fig. 5 is a block diagram showing an example of a hardware configuration of the therapy planning apparatus of Fig. 1.
[Fig. 6] Fig. 6 is a flowchart showing a particle therapy planning method according to the embodiment.
[Fig. 7] Fig. 7 is a flowchart showing a dose distribution calculation processing according to the embodiment.
[Fig. 8] Fig. 8 is a diagram showing an example of a 4D CT image used for dose distribution calculation according to the embodiment.
[Fig. 9] Fig. 9 is a perspective view showing an example of a discretization method of an irradiation amount during an irradiation position change in the continuous scanning irradiation according to the embodiment.
[Fig. 10] Fig. 10 is a diagram showing an example of a method for distributing the irradiation amount to each phase in the continuous scanning irradiation according to the embodiment.

### Description of Embodiments

Hereinafter, embodiments will be described with reference to the drawings. It should be noted that the embodiments described below do not limit the invention according to the claims, and all of the elements and combinations thereof described in the embodiments are not necessarily essential to the solution to the problem.

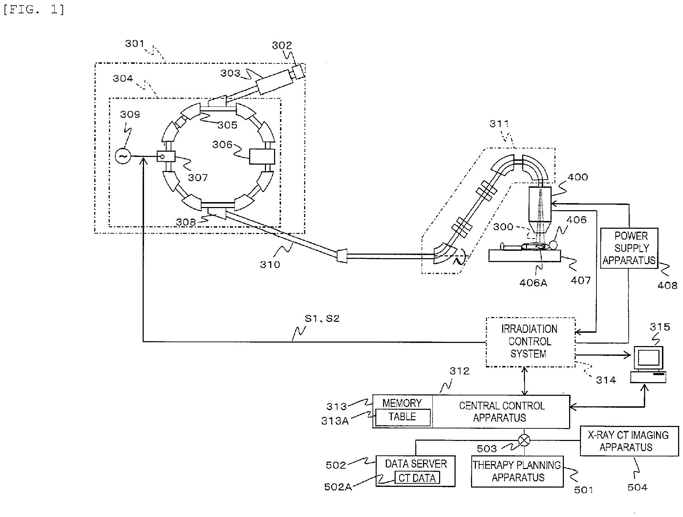

Fig. 1 is a block diagram showing a configuration of a particle therapy system to which a particle therapy planning apparatus is applied according to an embodiment.

In Fig. 1, the particle therapy system includes a charged particle beam generator 301, a high-energy beam transport system 310, a rotating irradiation apparatus 311, a central control apparatus 312, a memory 313, an irradiation control system 314, a display apparatus 315, an irradiation field forming apparatus (irradiation apparatus) 400, a bed 407, and a power supply apparatus 408. The central control apparatus 312, a therapy planning apparatus 501, a data server 502, and an X-ray CT imaging apparatus 504 are connected via a network 503. An X-ray fluoroscopy apparatus may be used instead of the X-ray CT imaging apparatus 504.

The charged particle beam generator 301 generates an accelerated particle beam 300 until a predetermined energy is reached. The charged particle beam generator 301 includes an ion source 302, a pre-accelerator 303, and a particle beam acceleration apparatus 304. The particle beam acceleration apparatus 304 includes, on a circumference orbit thereof, bending magnets 305, an acceleration apparatus 306, an extraction radiofrequency acceleration apparatus 307, an extraction deflector 308, and a quadrupole magnet (not shown) . A radiofrequency power supply 309 is connected to the extraction radiofrequency acceleration apparatus 307.

Although a synchrotron particle beam acceleration apparatus is assumed as the particle beam acceleration apparatus 304 in Fig. 1, another particle beam acceleration apparatus such as a cyclotron may be used as the particle beam acceleration apparatus 304.

The high-energy beam transport system 310 connects the particle beam acceleration apparatus 304 with the irradiation field forming apparatus 400. The rotating irradiation apparatus 311 adjusts an irradiation direction of the particle beam 300 to a patient 406. At this time, since the entire rotating irradiation apparatus 311 can rotate, the irradiation direction of the particle beam 300 can be adjusted in any direction around the bed 407, and the patient 406 can be irradiated with the particle beam 300 from any direction.

The central control apparatus 312 controls the irradiation control system 314 such that a dose distribution of the particle beam 300 is formed according to a therapy plan for the patient 406. The memory 313 holds data used for the control performed by the central control apparatus 312. For example, the memory 313 can hold a table 313A. A relationship between a deflection amount of the particle beam 300 and a current amount supplied to the irradiation field forming apparatus 400 can be set in the table 313A.

The irradiation control system 314 controls an irradiation amount, an irradiation position, and an irradiation direction of the particle beam 300. The display apparatus 315 displays a therapy progress status of the patient 406. The irradiation field forming apparatus 400 shapes the particle beam 300 with which the patient 406 is to be irradiated. A structure of the irradiation field forming apparatus 400 varies according to different irradiation methods. Typical irradiation methods include a scattering method and a scanning method. This embodiment is directed to the scanning method. According to the scanning method, a target 406A is three-dimensionally scanned and irradiated with the fine particle beam 300 transported from the high-energy beam transport system 310. The dose distribution of the particle beam 300 is only formed for the target 406A.

The scanning method has two ways of scanning, including discrete scanning irradiation and continuous scanning irradiation. In the discrete scanning irradiation, irradiation of the particle beam 300 is performed only when the irradiation position is not being changed, and irradiation of the particle beam 300 is stopped when the irradiation position is being changed. In the continuous scanning irradiation, the irradiation position is changed while irradiation of the particle beam 300 is performed continuously without stopping. This embodiment is directed to the continuous scanning irradiation.

The bed 407 holds the patient 406 in a lying state. An angle of a sleeping stage of the bed 407 can be adjusted. The power supply apparatus 408 supplies the irradiation field forming apparatus 400 with a current to perform scanning with the particle beam 300. The therapy planning apparatus 501 creates the therapy plan for the patient 406 using the particle beam 300. At this time, the therapy planning apparatus 501 calculates the irradiation amount of the particle beam 300 during an irradiation position change in the continuous scanning irradiation. Then, the therapy planning apparatus 501 calculates a dose distribution of the particle beam 300 with respect to a moving target 406A based on the irradiation amount of the particle beam during the irradiation position change.

The data server 502 holds data used in the therapy plan for the patient 406. For example, the data server 502 can hold CT data 502A. The CT data 502A includes a 4D CT image of the patient 406. The 4D CT image is a series of 3D CT images of the target 406A imaged at different time points. The 4D CT image reflects a state of the moving target 406A at different time points. The X-ray CT imaging apparatus 504 generates the 4D CT image.

Before a therapy with the particle beam 300 is applied to the patient 406, the therapy plan is prepared by the therapy planning apparatus 501 for the patient 406. When the therapy plan for the patient 406 is prepared, the 4D CT image of the patient 406 is generated by the X-ray CT imaging apparatus 504 and stored in the data server 502.

Next, the therapy planning apparatus 501 obtains the 4D CT image of the patient 406 from the data server 502. Then, the therapy planning apparatus 501 discretizes the irradiation amount of the particle beam 300 during the irradiation position change for calculation. Further, the therapy planning apparatus 501 associates the series of 3D CT images included in the 4D CT image with elapsed time information from a start to a completion of the irradiation of the particle beam 300. Furthermore, based on the series of 3D CT images and the elapsed time information that are associated, the therapy planning apparatus 501 distributes the irradiation amount during the irradiation position change that are discretized for calculation to the 3D CT images, and calculates a dose distribution of particle beam 300 on the 3D CT images to which the irradiation amount is distributed.

The therapy planning apparatus 501 calculates corresponding positions between the 3D CT images based on non-rigid registration. Then, the therapy planning apparatus 501 integrates a dose distribution formed for each of the 3D CT images for each of the corresponding positions between the 3D CT images from the start to the completion of the irradiation of the particle beam 300, thereby calculating a dose distribution after the completion of the irradiation of the particle beam 300.

When the therapy plan for the patient 406 is prepared, a particle therapy of the patient 406 is performed according to the therapy plan. In the particle therapy, the irradiation amount, the irradiation position, the irradiation direction, and the irradiation energy of the particle beam 300 to the target 406A are controlled according to the dose distribution of the particle beam 300 prepared by the therapy planning apparatus 501.

Hereinafter, a process from generation of the particle beam 300 from the charged particle beam generator 301, using a synchrotron particle beam acceleration apparatus 304, to extraction of the particle beam 300 to the target 406A will be described.

The particle supplied by the ion source 302 is accelerated by the pre-accelerator 303 and sent to the particle beam acceleration apparatus 304. The particle sequentially sent from the pre-accelerator 303 to the particle beam acceleration apparatus 304 circulates in the particle beam acceleration apparatus 304, and the particle beam accelerated to a predetermined energy is generated. At this time, a radiofrequency is applied to a radiofrequency acceleration cavity (not shown) disposed in the acceleration apparatus 306 in synchronization with a period when the particle beam passes through the acceleration apparatus 306, and the particle beam is accelerated until a predetermined energy is reached. The bending magnets 305 control a magnetic field in accordance with the energy of the particle beam such that an orbit along which the particle beam circulates in the particle beam acceleration apparatus 304 is constant.

When the particle beam is accelerated to a predetermined energy (e.g., 70 MeV to 250 MeV), the central control apparatus 312 outputs an extraction start signal S1 via the irradiation control system 314. At this time, radiofrequency power from the radiofrequency power supply 309 is applied to the particle beam circulating in the particle beam acceleration apparatus 304 via an extraction radiofrequency electrode disposed in the extraction radiofrequency acceleration apparatus 307. The particle beam is extracted from the particle beam acceleration apparatus 304.

The particle beam extracted from the particle beam acceleration apparatus 304 enters the high-energy beam transport system 310 via the extraction deflector 308, and is guided to the irradiation field forming apparatus 400 disposed in the rotating irradiation apparatus 311 via the high-energy beam transport system 310. The irradiation field forming apparatus 400 performs the continuous scanning irradiation in which the irradiation position is changed while the irradiation of the particle beam is performed. That is, the irradiation field forming apparatus 400 continuously changes an excitation amount of the magnets, and performs the irradiation of the particle beam while moving the particle beam so as to pass the particle beam through the entire irradiation field.

At this time, the central control apparatus 312 controls the irradiation control system 314 such that a dose distribution calculated by the therapy planning apparatus 501 is formed. Monitor information regarding the dose and position of the particle beam 300 is input from the irradiation field forming apparatus 400 to the irradiation control system 314. Then, the irradiation control system 314 controls an acceleration energy of the particle beam acceleration apparatus 304, a rotation angle of the rotating irradiation apparatus 311, and a current amount of the irradiation field forming apparatus 400 while monitoring the dose and position of the particle beam 300, and causes the dose distribution instructed by the central control apparatus 312 to be formed with respect to the target 406A. When the dose distribution instructed by the central control apparatus 312 is formed with respect to the target 406A, the central control apparatus 312 outputs an extraction stop signal S2 via the irradiation control system 314.

Here, since the therapy planning apparatus 501 calculates the dose distribution of the particle beam 300 with respect to the moving target 406A based on the irradiation amount of the particle beam 300 during the irradiation position change, the dose between irradiation positions during a movement can be reflected on the dose distribution. Therefore, the dose distribution regarding the continuously scanned target 406A can be accurately calculated based on the 4D CT image.

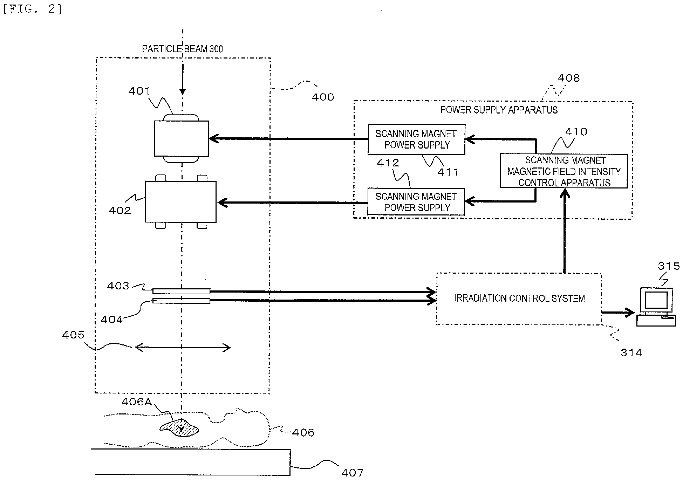

Fig. 2 is a block diagram showing configurations of the irradiation field forming apparatus and the power supply apparatus of Fig. 1.

In Fig. 2, the irradiation field forming apparatus 400 includes two scanning magnets 401 and 402, a dose monitor 403, and a beam position monitor 404 from an upstream side. The power supply apparatus 408 includes a scanning magnet magnetic field intensity control apparatus 410 and scanning magnets power supplies 411 and 412. The scanning magnets 401 and 402 generate magnetic force lines in a direction perpendicular to a traveling direction of the particle beam 300.

The particle beam 300 transported from the charged particle beam generator 301 through the high-energy beam transport system 310 enters the irradiation field forming apparatus 400. At this time, the irradiation control system 314 controls a current amount flowing to the scanning magnets 401 and 402 via the scanning magnet magnetic field intensity control apparatus 410. When the current is supplied to the scanning magnets 401 and 402, a magnetic field corresponding to a current amount is excited, and the deflection amount of the particle beam 300 is set. Since the irradiation control system 314 refers to the table 313A held in the memory 313, a relationship between the deflection amount of the particle beam 300 and the current amount can be obtained.

At this time, the scanning magnet 401 deflects the particle beam 300 in a scan direction 405, and the scanning magnet 402 deflects the particle beam 300 in a direction perpendicular to the scan direction 405. Accordingly, the irradiation field forming apparatus 400 can move the particle beam 300 to any position in a plane perpendicular to the traveling direction of the particle beam 300, and thereby the continuous scanning irradiation to fill an inside of the target 406A can be implemented.

During the continuous scanning irradiation, the dose monitor 403 measures the amount of the particle beam 300 that passes through the dose monitor 403, and the beam position monitor 404 measures the position where the particle beam 300 passes. The irradiation control system 314 supervises whether the position according to the therapy plan is irradiated with the particle beam 300 of the dose according to the therapy plan based on measurement values of the dose monitor 403 and the beam position monitor 404.

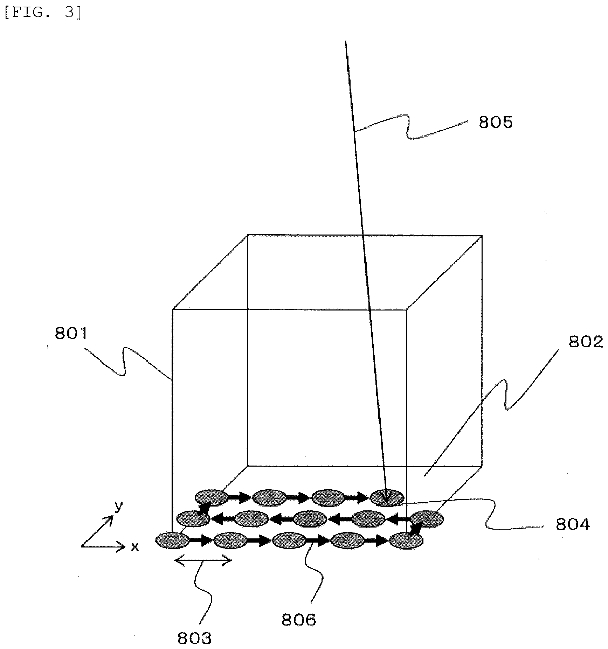

Fig. 3 is a perspective view showing an example of setting an irradiation position in the continuous scanning irradiation according to the embodiment. Fig. 3 shows an example in which a cubic target 801 is irradiated.

In Fig. 3, the particle beam is stopped at a certain position in the traveling direction, and most of the energy is given to the stopping position. Therefore, the energy is adjusted such that a depth at which the particle beam is stopped is within a target region.

Selected in the example of Fig. 3 is the particle beam stopping near a plane 802 and having an energy same as the irradiation energy received by the plane 802. Irradiation spots 804 set as irradiation positions are provided on the plane 802 at spot intervals 803. When one irradiation spot 804 is irradiated with a specified amount of the particle beam, the particle beam moves to a next irradiation spot 804, and the irradiation spot 804 as a movement destination is irradiated with the particle beam. A scan path 806 of the particle beam can be set, for example, as a zigzag path. At this time, after the particle beam moves from one end to the other end of the plane 802 along an x axis, the particle beam moves along a y axis only by one spot interval 803, and then the movement of the particle beam from one end to the other end of the plane 802 along the x axis can be repeated.

A dose distribution of each irradiation spot 804 may be given by a Gaussian distribution. At this time, the spot interval 803 can be set such that a dose distribution of the entire plane 802 is flattened by superimposing the Gaussian distributions of the irradiation spots 804.

An irradiation amount of the particle beam during the movement is also measured. When a sum of an irradiation amount of the particle beam during a movement and an irradiation amount of the particle beam in a state of being stopped at a next irradiation spot 804 reaches a specified amount, the particle beam further moves to a next irradiation spot 804. The irradiation spot 804 is irradiated with the particle beam passing through a locus 805 generated by irradiating the irradiation spot 804. When sequential irradiation of the irradiation spots 804 that are disposed inside the target 801 and irradiated with the same energy is completed, the depth at which the particle beam is stopped inside the target 801 is changed so as to irradiate another depth position inside the target 801.

In another method of the continuous scanning irradiation, instead of stopping at a predetermined irradiation position, the dose distribution can also be formed by scanning at a constant velocity so as to adjust intensity of the particle beam. In addition, instead of changing the intensity of the particle beam, the dose distribution that covers the target 801 can be formed by adjusting a scan velocity or by adjusting both the intensity of the particle beam and the scan velocity.

In order to change the depth at which the particle beam is stopped, the energy of the particle beam with which the target 801 is irradiated is changed. One method of changing the energy is to change setting of the particle beam acceleration apparatus 304, that is, the synchrotron in this embodiment. The particle is accelerated to the energy set in the synchrotron, and the energy entering the target 801 can be changed by changing the set energy value.

In this case, since the energy extracted from the synchrotron is changed, the energy when passing through the high-energy beam transport system 310 is also changed, and setting of the high-energy beam transport system 310 is also required to be changed. In the case of the synchrotron, a time of about 1 second is required for energy change.

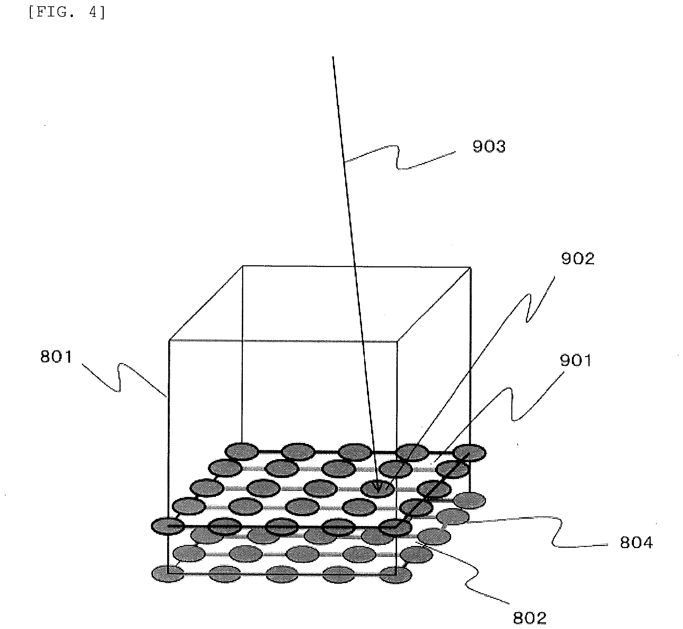

Fig. 4 is a perspective view showing an example of setting of an irradiation position when the energy is changed in the continuous scanning irradiation according to the embodiment.

In the example of Fig. 4, a particle beam having energy lower than the energy used in Fig. 3 is used. Therefore, the particle beam is stopped at a position shallower than the plane 802 of Fig. 3. This stopping position is represented by a plane 901 irradiated with the same energy. An irradiation spot 902 corresponding to the particle beam having the same energy is irradiated with the particle beam passing through a locus 903 generated by irradiating the irradiation spot 902.

Another method of changing the energy of the particle beam is to insert a range shifter (not shown) into the irradiation field forming apparatus 400. A thickness of the range shifter is selected according to the energy to be changed. For selection of the thickness, a method using a plurality of range shifters having a plurality of thicknesses, or a wedge-shaped opposing range shifter may be used. Since the time required for the energy change in this method is only the time for inserting the range shifter, this method can be performed at a relative higher speed than that of changing the setting of the synchrotron.

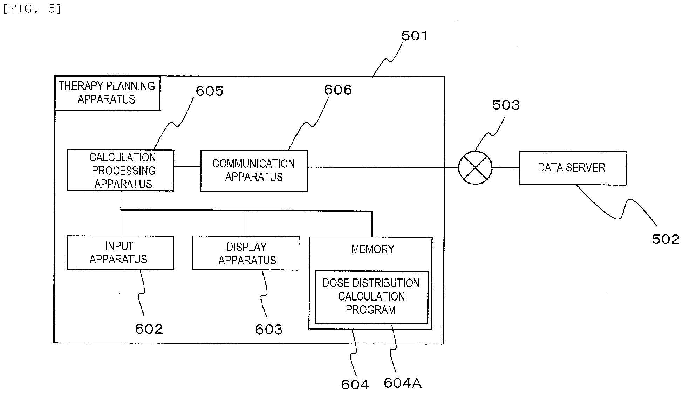

Fig 5 is a block diagram showing an example of a hardware configuration of the therapy planning apparatus of Fig. 1.

In Fig. 5, the therapy planning apparatus 501 includes an input apparatus 602, a display apparatus 603, a memory (storage apparatus) 604, a calculation processing apparatus (calculating apparatus) 605, and a communication apparatus 606. The calculation processing apparatus 605 is connected to the input apparatus 602, the display apparatus 603, the memory 604, and the communication apparatus 606. The input apparatus 602 inputs parameters for irradiation of the particle beam. An apparatus such as a mouse or a keyboard can be used as the input apparatus 602. The display apparatus 603 displays a therapy plan. An apparatus such as a liquid crystal display can be used as the display apparatus 603. A touch panel in which the input apparatus 602 and the display apparatus 603 are integrated may be used.

The memory 604 stores a program being executed by the calculation processing apparatus 605, and provides a work area for the calculation processing apparatus 605 to execute the program. A dose distribution calculation program 604A is stored in the memory 604. The dose distribution calculation program 604A may be software that can be installed in the therapy planning apparatus 501 or may be incorporated into the therapy planning apparatus 501 as firmware.

The calculation processing apparatus 605 is hardware that controls operation of the entire therapy planning apparatus 501. A processor, a microprocessor, or a central processing unit (CPU) can be used as the calculation processing apparatus 605. The communication apparatus 606 is hardware having a function of controlling communication with the outside.

The dose distribution calculation program 604A is executed by the calculation processing apparatus 605, and thereby dose distribution calculation can be performed. At this time, the calculation processing apparatus 605 can calculate the dose distribution of the particle beam with respect to the moving target based on the irradiation amount of the particle beam during the irradiation position change. Execution of the dose distribution calculation program 604A may be shared by a plurality of processors or computers. Alternatively, the calculation processing apparatus 605 may instruct all or part of the execution of the dose distribution calculation program 604A to a cloud computer or the like, and may receive an execution result thereof.

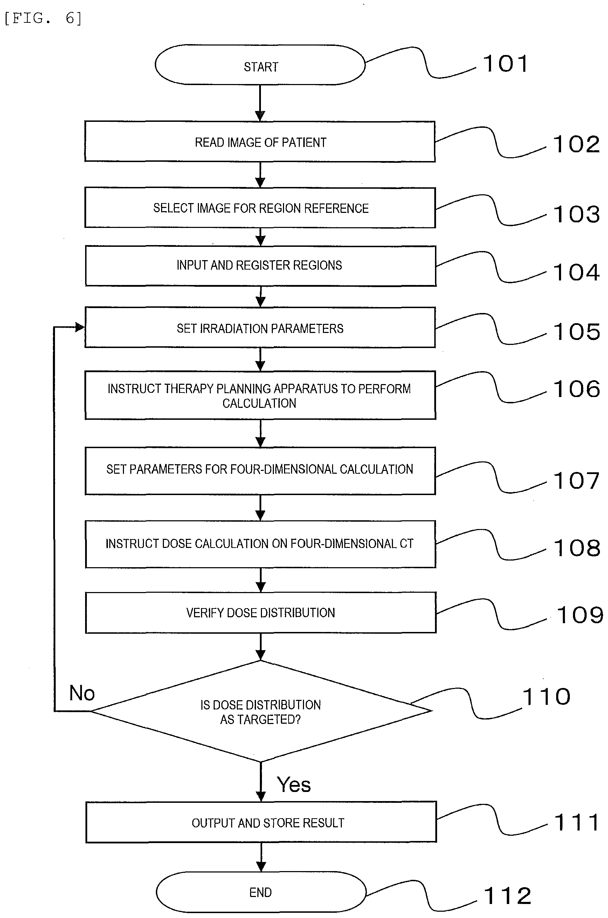

Fig. 6 is a flowchart showing a particle therapy planning method according to the embodiment.

In Fig. 6, images for the therapy plan are imaged prior to the particle therapy. CT images are the most commonly used for the therapy plan. The CT images are obtained by reconstructing three-dimensional data from perspective images obtained in a plurality of directions from the patient. Since a 4D CT image is used to create the therapy plan in this embodiment, the CT image captured here is also the 4D CT image. However, the 4D CT image referred to here is not limited to an image obtained by a special imaging method different from normal CT images, and represents a data set including a plurality of 3D CT images in a plurality of different states of the same patient.

The 4D CT image imaged by the X-ray CT imaging apparatus 504 of Fig. 1 is stored in the data server 502. The therapy planning apparatus 501 prepares the therapy plan using the 4D CT image. When preparation of the therapy plan starts (step 101), a technician (or doctor) who is an operator of the therapy planning apparatus 501 causes the therapy planning apparatus 501 to read target CT data from the data server 502 using the input apparatus 602. That is, the therapy planning apparatus 501 copies the 4D CT image from the data server 502 to the memory 604 through the network 503 connected to the communication apparatus 606 by the operation of the input apparatus 602 (step 102).

The 4D CT image includes a set of 3D CT images and holds movement information including translation, rotation, and deformation of the target region and a surrounding internal structure. In this embodiment, such a series of 3D CT images that hold information regarding changes in a region irradiated with the particle beam are referred to as the 4D CT image. Each of the 3D CT images constituting the 4D CT image has a time relationship. In a case where the movement of the target changes periodically, each of the 3D CT images shows a state of a region at different time points during a certain movement cycle (e.g., respiration or heartbeat cycle of the patient). At this time, a phase can be assigned to each of the 3D CT images according to the time relationships of these 3D CT images.

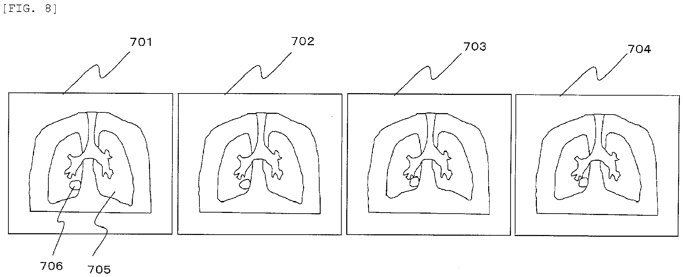

Fig. 8 is a diagram showing an example of the 4D CT image used for dose distribution calculation according to the embodiment.

In Fig. 8, it is assumed that a tumor is present in the lung 705 of the patient 406, and a therapy plan for the tumor with a particle therapy is prepared. At this time, a 4D CT image of a chest region of the patient 406 is obtained by the X-ray CT imaging apparatus 504. For example, a series of four 3D CT images 701 to 704 are obtained as the 4D CT image. In Fig. 8, the four 3D CT images 701 to 704 are shown in the form of two-dimensional CT images for convenience.

The 3D CT images 701 to 704 each show the lung 705 and a target region 706 of the patient 406. The target region 706 is a region to be designated as a target by the operator. The 3D CT images 701 to 704 represent states imaged at four time points T1 to T4 in a respiratory cycle of the patient. The 3D CT images 701 to 704 are associated with information about what state the respiratory cycle is in.

For example, one respiratory cycle is divided into a fully exhaled state, a fully inhaled state, and intermediate states thereof . A phase is assigned to each of the 3D CT images 701 to 704. In this example, the 4D CT image includes a single movement (respiration) cycle, and may be obtained over a plurality of movement cycles. The set of 3D CT images are not limited to one cycle as long as time and state information are associated with each 3D CT image.

Referring back to Fig. 6, when the reading of the 4D CT image from the data server 502 to the memory 604 is completed, the 4D CT image is displayed on the display apparatus 603. The operator selects a 3D CT image that refers to the region to be designated as the target as an image for region reference (step 103).

Next, while checking the 3D CT images displayed on the display apparatus 603, the operator uses the input apparatus 602 to input a target region for a slice of each of the 3D CT images, that is, a two-dimensional CT image. The target region to be input is a region which is determined to be irradiated with a sufficient amount of the particle beam due to the presence or possible presence of a tumor cell. In a case where there are other regions requiring evaluation or control, such as important organs whose irradiation dose should be controlled to a minimum in the vicinity of the target region, the operator designates regions such as those important organs in the same manner.

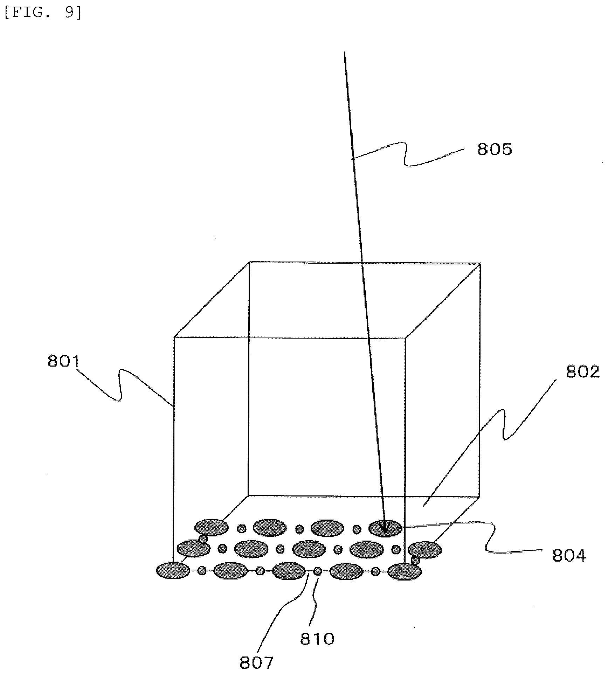

Here, region designation may be performed in all of the individual 3D CT images included in the 4D CT image, but only a 3D CT image in one state included in the 4D CT image can be used. For example, the operator may select the 3D CT image 701 of the fully exhaled state from the 3D CT images 701 to 704 in Fig. 9, and designate the target region 706 on the 3D CT image 701.

Alternatively, one 3D CT image can be synthesized from all the 3D CT images, and the target region can be designated on the 3D CT image. For example, one set of synthesized CT images can be obtained by comparing CT values of points representing a same position of a plurality of 3D CT images and selecting one point having the highest luminance value out of all points. In addition, the target region may be designated on images of a different modality represented by Magnetic Resonance Imaging (MRI).

In a case where the target region or a region of an important organ is input only on one 3D CT image, the region on each of the 3D CT images included in the 4D CT image can be determined using a non-rigid registration technique. The non-rigid registration technique may require correction performed by the operator, but after a moving model (a movement direction and size of each point) is defined, it is possible to designate another image region corresponding to a shape of the region in a certain image, including deformation of the target region. Accordingly, it is possible to save the time and effort for the operator to specify the region for each of the 3D CT images included in the 4D CT image, and reduce the workload of the operator.

When the region input is finished for all the 3D CT images, the operator instructs registration of the input regions. The regions input by the operator are stored as three-dimensional position information in the memory 604 by instructing the registration (step 104). The position information about the regions can also be stored in the data server 502, and the therapy planning apparatus 501 can read the position information input in the past together with the 3D CT images from the data server 502.

Next, the operator creates the therapy plan including information about the position and the energy of the particle beams with which the registered target regions are to be irradiated. Although the 4D CT image includes the plurality of 3D CT images that hold information about a target region change, the therapy plan based on the 3D CT images is prepared referring to one specific 3D CT image. The 3D CT image for which the region is input in step 104 is generally selected as the 3D CT image that is referred to. In addition, a 3D CT image corresponding to another phase in the 4D CT image may be referred to, or an ordinary 3D CT image imaged separately from the 4D CT image for the same patient may be referred to.

The following operations are performed based on the selected 3D CT image.

The operator sets irradiation parameters necessary for calculating the dose distribution on the selected 3D CT image (step 105). The irradiation parameters to be set by the operator include the irradiation direction. The particle therapy system to which this embodiment is applied can irradiate, with the particle beam 300, the patient 406 in any direction by selecting an angle between the rotating irradiation apparatus 311 and the bed 407. A plurality of irradiation directions can be set for one target 406A. Generally, the vicinity of the center of the target region 706 is positioned so as to coincide with an isocenter (a rotational center position of the rotating irradiation apparatus 311).

Other irradiation parameters to be set by the operator include a dose value (prescription dose) with which the regions registered in step 104 are to be irradiated. The prescription dose includes a dose with which the target 406A is to be irradiated and a maximum dose to be avoided for important organs. When the above irradiation parameters are set, the therapy planning apparatus 501 automatically performs dose calculation according to an instruction from the operator (step 106).

Details of the processing regarding the dose calculation performed by the therapy planning apparatus 501 based on the specific 3D CT image will be described below.

The therapy planning apparatus 501 determines the scan path and the irradiation positions of the particle beam. The irradiation positions are set so as to cover the target region. In a case where a plurality of directions are designated as the irradiation direction (angles between the rotating irradiation apparatus 311 and the bed 407), the therapy planning apparatus 501 performs the same processing in each of the irradiation directions.

When all the irradiation positions are determined, the therapy planning apparatus 501 starts optimization calculation of the irradiation amount. The therapy planning apparatus 501 calculates the irradiation amount such that the irradiation amount with which each spot determined as the irradiation position is irradiated approaches a target prescription dose set in step 105. In this calculation, an objective function that numerically expresses a deviation from the target dose that uses the irradiation amount for each spot as a parameter can be used. The objective function is defined to have a smaller value such that the dose distribution satisfies the target dose. The therapy planning apparatus 501 calculates an optimum irradiation amount by repeating calculation so as to search for an irradiation amount that minimizes the objective function. When the repeated calculation is completed, the irradiation amount necessary for each spot is finally determined.

Next, the therapy planning apparatus 501 causes the calculation processing apparatus 605 to calculate the dose distribution based on the finally obtained position and irradiation amount of each spot. If necessary, the therapy planning apparatus 501 causes the display apparatus 603 to display a calculation result of the dose distribution. The calculation result obtained at this stage is the calculation result with respect to the 3D CT image selected in step 103. Therefore, the calculation result obtained at this stage does not reflect information about the target region change such as movement, deformation, or rotation of the target during a period of performing irradiation with the particle beam.

The therapy planning apparatus 501 according to this embodiment can not only calculate the dose distribution obtained based on the created therapy plan only on the specific 3D CT image, but also calculate and display the dose distribution after integrating information about the 4D CT image, that is, information about the 3D CT images in a plurality of different states.

In order to calculate the dose distribution after integrating the information about the 4D CT image, the operator performs step 107 and the processing after step 107. At this time, the operator sets parameters for four-dimensional calculation (step 107), and instructs the dose calculation on four-dimensional CT (step 108).

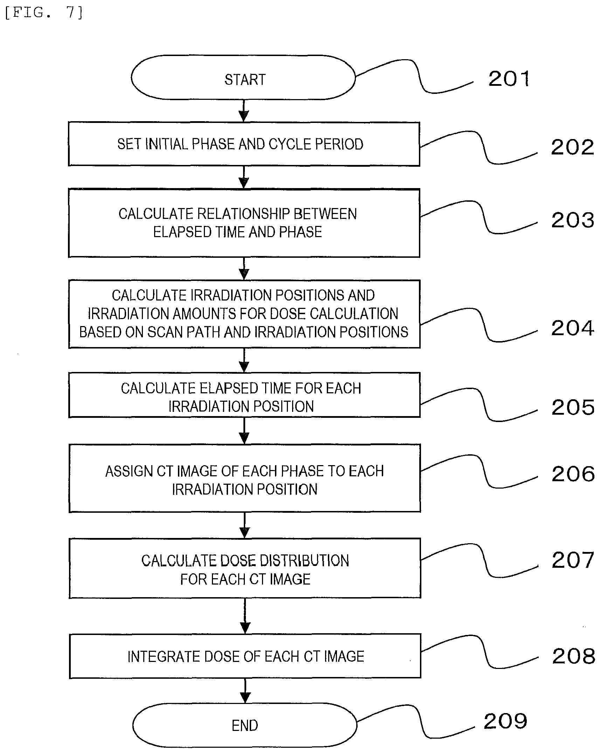

Hereinafter, a part corresponding to step 107 of Fig. 6 is shown in step 202 of Fig. 7, and a calculation processing flow of the therapy planning apparatus 501 started by an instruction of step 108 in Fig. 6 is shown in steps 203 to 209 of Fig. 7.

Fig. 7 is a flowchart showing a dose distribution calculation processing according to the embodiment.

In Fig. 7, the therapy planning apparatus 501 can select any movement state of a target volume at a time point starting the particle beam irradiation according to the therapy plan.

When the therapy plan in which the information about the 4D CT image is integrated is started (step 201), the operator designates a state (phase) when the particle beam irradiation is started (step 202). As described above, each 3D CT image included in the 4D CT image includes phase information corresponding to the target region change. For example, as shown in Fig. 9, in the case of a 4D CT image including information about the target region change caused by respiration, one respiratory cycle is divided into four states, i.e., a fully exhaled state, a fully inhaled state, and intermediate states thereof (phases). These four states are represented by the 3D CT images 701 to 704, respectively.

When the respiratory cycle is repeated in this order, the phase of each state can be designated with a real number between 0 and 1 such that the phase of the state of the 3D CT image 701 is 0, the phase of the state of the 3D CT image 702 is 0.25, the phase of the state of the 3D CT image 703 is 0.5, the phase of the state of the 3D CT image 704 is 0.75, and then the respiratory cycle returns the 3D CT image 701 with a phase of 1.0. Since designation of the phase with a real number between 0 and 1 is an example, if there is an index that is easier to understand for the operator, such index may be adopted.

The phase of each 3D CT image included in the 4D CT image may be obtained in advance by a device that images the 4D CT image, or may be set by the operator using the input apparatus 602. If the operator can set the phases regarding the target region change with respect to the series of 3D CT images using the input apparatus 602, the images can be used by the therapy planning apparatus 501 even not obtained through a special imaging method.

The calculation processing apparatus 605 assigns a phase corresponding to the elapsed time from the start of the irradiation, according to the phase and the movement cycle at the start of the irradiation input in step 202 (step 203). That is, the calculation processing apparatus 605 associates a phase set for each of the series of three-dimensional images that constitute the 4D CT image with the elapsed time information from the start to the completion of the particle therapy according to the therapy plan. Accordingly, the calculation processing apparatus 605 can refer to which phase state the patient is in at a given time from the start of the irradiation.

For this association, information about a period of the movement cycle included in the 4D CT image, that is, a target region change period is necessary. In a case where the period is known from information obtained in imaging the 4D CT image, the information can be used. In a case where the period is not known from the information obtained in imaging the 4D CT image, the operator can input a typical value in step 202, for example, a value of about several seconds in case of a respiratory cycle, via the input apparatus 602.

Next, the calculation processing apparatus 605 sets calculation spots that discretize the irradiation amount during the irradiation position change for calculation (step 204). In the continuous scanning irradiation, the particle beam irradiation is continued even during the irradiation position change. Here, since the calculation spots are set, it is possible to discretize the doses continuously irradiated during the irradiation position change, and to accurately reflect the doses during the irradiation position change on a target dose distribution.

Fig. 9 is a perspective view showing an example of a discretization method of the irradiation amount during the irradiation position change in the continuous scanning irradiation according to the embodiment.

In Fig. 9, the calculation processing apparatus 605 sets a calculation spot 810 between irradiation spots 804 in order to discretize the irradiation amount during the irradiation position change for calculation. The calculation accuracy is improved as the number of the calculation spots 810 increases, but the calculation time increases, so that it is necessary to set an appropriate number. The longer a distance between the irradiation spots 804, the larger the irradiation amount is during the irradiation position change, which causes a decrease in the calculation accuracy. Therefore, setting a large number of the calculation spots 810 is effective in improving the calculation accuracy. Further, in the continuous scanning irradiation, since current intensity of the particle beam during the irradiation is designated, it is effective to increase the number of the calculation spots 810 as the current intensity increases.

The calculation accuracy of the dose distribution on the 4D CT image also depends on the movement in the patient. For example, in a case where the respiratory cycle of the patient is short or the movement magnitude of the target is large, the movement of the target is fast, and the calculation accuracy is prone to decrease. Therefore, it is effective to register more calculation spots 810 as the respiratory cycle is shorter and the movement magnitude of the target is larger. The information registered in step 202 can be used as the respiratory cycle. The movement magnitude of the target can be determined based on a movement amount of a gravity center of the region registered in step 104.

The calculation spot 810 is set on the scan path 807. The irradiation amount of the calculation spot 810 can be calculated as a product of the current intensity and a scan time to scan along a scan path length substituted by the calculation spot 810. Further, the irradiation amount with which the irradiation spot 804 is to be irradiated next is subtracted only by the irradiation amount of the calculation spot 810 determined here.

Referring back to Fig. 7, the calculation processing apparatus 605 simulates the movement of the particle therapy system from the start to the completion of the particle beam irradiation, and calculates the elapsed time from the start of the particle beam irradiation for each irradiation spot and calculation spot (step 205). Gated irradiation can also be considered for the movement of the particle therapy system. For example, the gated irradiation can be considered by limiting the phase at which the particle beam irradiation is performed. The gated irradiation is a method of reducing the influence of the movement of the target on the dose distribution by measuring the movement of a body surface or the movement of the target itself, and performing the particle beam irradiation only in a predetermined state.

Next, the calculation processing apparatus 605 groups all the irradiation spots and calculation spots into each phase (step 206). Then, based on a relationship between the phase of the 3D CT image and a phase of the irradiation spot, the irradiation amount assigned to the irradiation spot is distributed to the 3D CT image. Further, based on the relationship between the phase of the 3D CT image and a phase of the calculation spot, the irradiation amount assigned to the calculation spot is distributed to the 3D CT image.

This phase is determined from step 203 and step 205 in Fig. 7. That is, the phase at which a spot is irradiated can be determined from the relationship calculated in step 203 between the phase of the 4D CT image and the elapsed time from the start of irradiation, and from the elapsed time calculated in step 205 from the start of the irradiation at the time when the spot is irradiated.

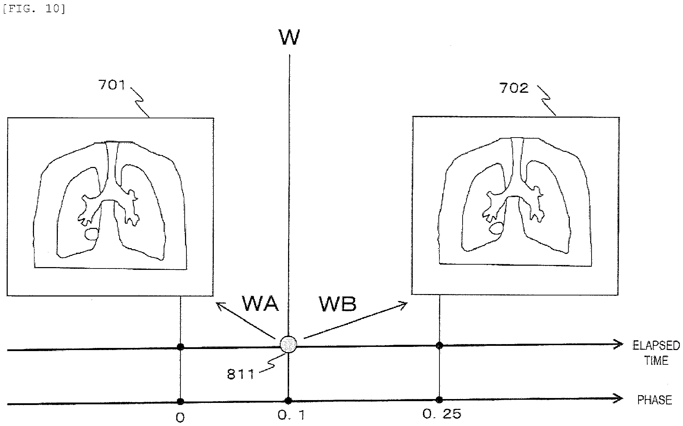

Fig. 10 is a diagram showing an example of a method for distributing an irradiation amount to each phase in the continuous scanning irradiation according to the embodiment.

In Fig. 10, for example, groups corresponding to the 3D CT images 701 to 704 with respect to the 4D CT image in Fig. 8 are denoted by A, B, C and D, respectively. Here, attention is paid to one spot 811 among all irradiation spots and calculation spots. At this time, for example, an irradiation amount of the spot 811 is set to W, and a phase at the time of irradiation of the spot 811 is set to 0.1. Phases before and after this spot are group A and group B, and respective phases of the groups are 0 and 0.25.

The calculation processing apparatus 605 linearly distributes the irradiation amount W of the spot 811 to the group A and the group B. At this time, the calculation processing apparatus 605 distributes an irradiation amount WA = W ((0.25-0.1) / 0.25) to the group A, and distributes an irradiation amount WB = W (0.1 / 0.25) to the group B. Here, since the irradiation amount W of the spot 811 is distributed to the group A and the group B, it is possible to cope with the continuous movement of the target between the phase 0 and the phase 0.25. Since the above-described distribution is repeated in the same manner for all the spots, an irradiation amount is assigned to the 3D CT images from 701 to 704 of each phase for each irradiation position.

Since one spot is divided into a preceding group and a succeeding group, the number of spots to be calculated is doubled. Here, although a distribution ratio is assumed to be linear, the distribution ratio may be weighted and distributed to a specific group. The distribution ratio is not limited to the above method.

Although Fig. 10 shows a method for distributing the irradiation amount W of the spot 811 to the 3D CT images 701 and 702, the calculation processing apparatus 605 may generate a 3D CT intermediate image having a phase between the phase 0 of the 3D CT image 701 and the phase 0.25 of the 3D CT image 702 by interpolating the 3D CT images 701 and 702. Then, based on a relationship between the phase of the spot 811 and the phase of the 3D CT intermediate image, the calculation processing apparatus 605 may distribute all or part of the irradiation amount W of the spot 811 to the 3D CT intermediate image.

Referring back to Fig. 7, the calculation processing apparatus 605 calculates a dose distribution for each group (step 207). That is, the calculation processing apparatus 605 calculates a dose distribution of the spots belonging to each group based on a 3D CT image corresponding to the group. For example, a dose distribution of the spots belonging to the group A is calculated based on the 3D CT image 701. The calculation results of the dose distribution are integrated for each group.

Here, as shown in Fig. 9, an irradiation amount on the continuous scan path during the irradiation position change can be expressed by a Gaussian distribution on the calculation spot 810 by setting the calculation spot 810 between the irradiation spots 804. Therefore, the dose distribution can be calculated by superimposing the Gaussian distribution of each spot distributed to each 3D CT image.

Next, the calculation processing apparatus 605 integrates all the dose distributions calculated in step 207 (step 208). The 3D CT images 701 to 704 represent different states in the body. Therefore, the calculation processing apparatus 605 determines a corresponding position between the 3D CT images 701 to 704 by the non-rigid registration. At this time, it is necessary to determine a 3D CT image serving as a reference for integrating the dose distributions.

For example, in a case where the 3D CT image 701 is set as a reference, calculation points of the 3D CT images 702 to 704 corresponding to a calculation point jA in the 3D CT image 701 are denoted by jB, jC, and jD, respectively. The calculation points jB, jC, and jD are calculated by the non-rigid registration. The calculation processing apparatus 605 can determine a final dose value at the calculation point jA by integrating a dose value at the calculation point jA calculated as the group A in step 206 and dose values at the calculation points jB, jC, and jD calculated as the groups B, C, and D, respectively. The calculation processing apparatus 605 performs this calculation for all the calculation points. After calculating the dose distribution for each group, the calculation processing apparatus 605 ends the processing (step 209).

Through the above processing, the calculation processing apparatus 605 can accurately calculate a final dose distribution with respect to the moving target during the continuous scanning irradiation. After calculating the final dose distribution, the calculation processing apparatus 605 causes the display apparatus 603 to display the dose distribution.

It should be noted that the dose distribution calculation program 604A shown in Fig. 5 may cause the calculation processing apparatus 605 to perform all of the processing of steps 203 to 208 in Fig. 7, or may cause the calculation processing apparatus 605 to perform the processing of steps 203 to 207 in Fig. 7, and may cause another program to perform the processing of step 208 in Fig. 7.

Referring back to Fig. 6, the operator evaluates the dose distribution formed when the particle beam irradiation is completed (step 109). At this time, the operator determines whether the dose distribution satisfies a target condition, or the degree of coincidence between the dose distributions formed when the particle beam irradiation is completed and a target dose distribution. It should be noted that such evaluation may be performed by artificial intelligence such as a neural network.

For evaluation, the operator can change initial phases designated in step 107 and perform calculation again. Alternatively, after several initial phases are selected and calculated automatically, a method of calculating an average value of deviations from target distributions can also be prepared. The operator can evaluate average influence of the target region change on planned dose distributions by calculating an average value of deviations between the dose distributions when a plurality of initial phases are set and the target dose distributions.

In a case where it is determined that the evaluation result of the finally formed dose distribution is not a desired dose distribution for the operator, the processing returns to step 105, and the irradiation parameters are reset. The irradiation parameters to be changed include the irradiation direction and the prescribed dose. In calculation using the 4D CT image, since the scan path and repeating irradiation times also influence the dose distribution, these values can be set as well.

After the setting of the irradiation parameters is changed, the calculation using the 4D CT image is repeated until the dose distribution is the target dose distribution (step 110). When the target dose distribution is obtained, the preparation of the therapy plan is completed. Irradiation conditions obtained in this therapy plan are stored in the data server 502 via the network 503 (step 111). When the obtained irradiation conditions are stored, the processing is completed (step 112).

As described above, the therapy planning apparatus 501 according to this embodiment can reflect the irradiation amount during the irradiation position change of the particle beam on the dose distributions on the 4D CT image. Therefore, an influence of the moving target on the dose distribution during the continuous scanning irradiation can be reflected, and the calculation accuracy of the dose distribution finally formed with respect to the target can be improved.

Although this embodiment is described using a method of stopping and irradiating each spot in the continuous scanning irradiation, and a method of forming a dose distribution by continuously moving without stopping for each spot and adjusting the scan velocity and the particle beam intensity may also be applied. Even in this case, the dose distribution formed with respect to the moving target can be accurately calculated by registering an appropriate number of calculation spots and calculating the dose distribution on the 4D CT images .

In this embodiment, the dose distribution is calculated using the 4D CT image imaged before the therapy plan is created. Meanwhile, the 4D CT image may be imaged just before the daily particle beam irradiation. At this time, an X-ray CT imaging apparatus placed in a treatment room can be used to the image 4D CT image, or an X-ray fluoroscopic apparatus capable of rotating around the patient may be used to image a four-dimensional cone-beam CT image.

The central control apparatus 312 included in the particle therapy system of Fig. 1 can accurately calculate the dose distribution just before the therapy by reflecting the irradiation amount of the particle beam during the irradiation position change on the dose distribution on the 4D CT image imaged just before the therapy. The central control apparatus 312 can accurately predict the dose distribution to be actually irradiated by using the 4D CT image imaged just before the therapy. The dose distribution just before the therapy may be calculated by the central control apparatus 312 included in the particle therapy system, or may be calculated by the therapy planning apparatus 501 by transferring the 4D CT image imaged just before the therapy to the therapy planning apparatus 501.

The central control apparatus 312 can also record the target position information and irradiation time of the irradiation spots during the particle beam irradiation, and calculate the dose distribution on the 4D CT image. At this time, irradiation time of the calculation spots is interpolated based on the recorded irradiation time of the irradiation spots. Then, the actual irradiated dose distribution can be accurately calculated by assigning the irradiation spots and the calculation spots to each phase of the 4D CT image, and calculating the dose distribution. In addition, when calculating the dose distributions on the 4D CT image, the central control apparatus 312 can obtain the elapsed time information from the start to the completion of the irradiation of the particle beam from an irradiation record of the particle beam.

### Reference Sign List

301 charged particle beam generator
302 ion source
303 pre-accelerator
304 particle beam acceleration apparatus
305 bending magnet
306 acceleration apparatus
307 extraction radiofrequency acceleration apparatus
308 extraction deflector
309 radiofrequency power supply
310 high-energy beam transport system
311 rotating irradiation apparatus
312 central control apparatus
313, 604 memory
314, 603 display apparatus
400 irradiation field forming apparatus
401, 402 scanning magnet
403 dose monitor
404 beam position monitor
405 scan direction
406 patient
406A, 801 target
407 bed
408 power supply apparatus
410 scanning magnet magnetic field intensity control apparatus
411, 412 scanning magnet power supply
501 therapy planning apparatus
502 data server
504 X-ray CT imaging apparatus
602 input apparatus
605 calculation processing apparatus
606 communication apparatus

## Claims

1. A particle therapy planning apparatus comprising:
a calculation processing apparatus (605),
wherein the calculation processing apparatus (605) is configured to
calculate an irradiation amount of a particle beam during an irradiation position change in continuous scanning irradiation, and
calculate a dose distribution of the particle beam with respect to a moving target (406A) based on the irradiation amount of the particle beam during the irradiation position change.

2. The particle therapy planning apparatus according to claim 1, wherein the calculation processing apparatus (605) is configured to
obtain a 4D CT image that is a series of 3D CT images of the target (406A) imaged at different time points,
discretize the irradiation amount of the particle beam during the irradiation position change for calculation,
associate the series of 3D CT images with elapsed time information from a start to a completion of irradiation of the particle beam,
interpolate information regarding a target region change for each irradiation position of the particle beam based on the series of 3D CT images and the elapsed time information that are associated, and
calculate the dose distribution of the particle beam based on the irradiation amount discretized for the calculation and information regarding the target region change obtained by the interpolating.

3. The particle therapy planning apparatus according to claim 1, wherein the calculation processing apparatus (605) is configured to
obtain a 4D CT image that is a series of 3D CT images of the target (406A) imaged at different time points,
discretize the irradiation amount of the particle beam during the irradiation position change for calculation,
associate the series of 3D CT images with elapsed time information from a start to a completion of irradiation of the particle beam,
distribute the irradiation amount discretized for the calculation to the 3D CT images based on the series of 3D CT images and the elapsed time information that are associated, and
calculate the dose distribution of the particle beam on the 3D CT images to which the irradiation amount is distributed.

4. The particle therapy planning apparatus according to claim 1, wherein the calculation processing apparatus (605) is configured to
integrate a dose distribution formed for each of the 3D CT images from a start to a completion of irradiation of the particle beam, thereby calculating a dose distribution after the completion of the irradiation of the particle beam, and
display a dose distribution after the completion of the irradiation of the particle beam.

5. A particle therapy system comprising:
a control apparatus configured to calculate a dose distribution of a particle beam with respect to a moving target (406A) based on an irradiation amount of the particle beam during an irradiation position change;
a charged particle beam generator (301) configured to generate an accelerated particle beam until a predetermined energy is reached;
an irradiation control system configured to control an irradiation amount, an irradiation position, and an irradiation direction of the particle beam based on the dose distribution of the particle beam calculated by the control apparatus; and
an irradiation field forming apparatus (400) configured to shape the particle beam with which the target (406A) is to be irradiated based on control of the irradiation control system.

6. The particle therapy system according to claim 5, wherein the control apparatus is configured to
obtain a 4D CT image that is a series of 3D CT images of the target (406A) imaged at different time points,
discretize the irradiation amount of the particle beam during the irradiation position change for calculation,
associate the series of 3D CT images with elapsed time information from a start to a completion of irradiation of the particle beam,
interpolate information regarding a target region change for each irradiation position of the particle beam based on the series of 3D CT images and the elapsed time information that are associated, and
calculate the dose distribution of the particle beam based on the irradiation amount discretized for the calculation and information regarding the target region change obtained by the interpolating.

7. The particle therapy system according to claim 5, wherein the control apparatus is configured to
obtain a 4D CT image that is a series of 3D CT images of the target imaged at different time points,
discretize the irradiation amount of the particle beam during the irradiation position change for calculation,
associate the series of 3D CT images with elapsed time information from a start to a completion of irradiation of the particle beam,
distribute the irradiation amount discretized for the calculation to the 3D CT images based on the series of 3D CT images and the elapsed time information that are associated, and
calculate the dose distribution of the particle beam on the 3D CT images to which the irradiation amount is distributed.

8. The particle therapy system according to claim 7, wherein the control apparatus is configured to
obtain the 4D CT image generated just before the particle beam is irradiated,
calculate the dose distribution of the particle beam based on the 4D CT image, and
display the dose distribution of the particle beam.

9. The particle therapy system according to claim 7, wherein the control apparatus is configured to obtain the elapsed time information from the start to the completion of the irradiation of the particle beam from an irradiation record of the particle beam.

10. The particle therapy system according to claim 7, wherein the control apparatus is configured to
set a calculation spot on a scan path during the irradiation position change, the calculation spot discretizing the irradiation amount during the irradiation position change for the calculation, and
discretize the irradiation amount of the particle beam during the irradiation position change by assigning the irradiation amount of the particle beam on the scan path to the calculation spot.

11. The particle therapy system according to claim 10, wherein the control apparatus is configured to distribute the irradiation amount of the particle beam assigned to the calculation spot to the 3D CT images based on a position of the calculation spot.

12. The particle therapy system according to claim 11, wherein the control apparatus is configured to
assign a phase corresponding to a state of the target to the 3D CT images when a movement of the target (406A) changes periodically,
calculate a phase of the calculation spot based on the position of the calculation spot, and
distribute the irradiation amount of the particle beam assigned to the calculation spot to the 3D CT images based on a relationship between a phase of the 3D CT images and the phase of the calculation spot.

13. The particle therapy system according to claim 12, wherein the control apparatus is configured to distribute the irradiation amount of the particle beam to the 3D CT images having phases before and after the phase of the calculation spot.

14. The particle therapy system according to claim 13, wherein the control apparatus is configured to
calculate corresponding positions between the 3D CT images based on non-rigid registration, and
integrate a dose distribution formed for each of the 3D CT images for each of the corresponding positions between the 3D CT images from the start to the completion of the irradiation of the particle beam, thereby calculating a dose distribution after the completion of the irradiation of the particle beam.

15. A dose distribution calculation program causing a computer to
calculate an irradiation amount of a particle beam during an irradiation position change, and
calculate a dose distribution of the particle beam with respect to a moving target (406A) based on the irradiation amount of the particle beam during the irradiation position change.
